# EUROPEAN PATENT APPLICATION

(11) **EP 4 454 675 A1**
(43) Date of publication of application: **30.10.2024**
(21) Application number: 23170690.4
(22) Date of filing: 28.04.2023
(51) Int. Cl.: A61M 1/06

(54) **BREAST PUMP**

(71) Applicant: Medela Holding AG, 6340 Baar (CH)
(72) Inventor: PAWLOWSKI, Jakub, 6300 Zug (CH)
(74) Representative: Grünecker Patent- und Rechtsanwälte PartG mbB

(57) **Abstract**

The present invention relates to a breast pump (2) wearable inside a bra, including a breast shield (6) adapted to at least in part receive a breast of a lactating user, an aggregate (74) for generating suction for expressing milk from the breast, a milk container (14) providing a reservoir (46) for the expressed milk, an accelerometer (130) and a controller (82) operatively connected with the accelerometer (130) and provided for controlling the aggregate (74). To determine the volume of expressed milk within the milk container with higher accuracy, the present invention proposes a sensor (120) assigned to said reservoir (46) for determining the milk level within the reservoir (46), wherein said controller (82) is operatively connected with said sensor (120) and wherein said controller (82) is adapted to output a signal indicative of the risk of milk spillage from the reservoir (46) on the basis of the signals received form the accelerometer (130) and from said sensor (120).

## Description

The present invention relates to a breast pump and, in particular, a breast pump adapted to more accurately obtain and in particular output information relating to the amount of milk expressed and collected in a milk container of the breast pump.

WO 2018/229 504 A1 proposes an in-bra breast pump having a housing receiving an aggregate, a controller and a power source for energizing the aggregate and the control. A breast shield and a milk container are connected to the housing. In other words, all components of the breast pump are connectable to form an in-bra breast pump unit, which can be suspended by the bra of the user.

The breast pump disclosed in WO 2018/229 504 A1 has an accelerometer connected with a controller of the breast pump, which accelerometer infers the amount of movement or tilt angle during a pumping session. If the tilt angle excesses a threshold, the system warns or alerts the user of an imminent spillage, or provides the user with an alert to change position. Alternatively, the system may also stop pumping to prevent spillage, and once the tilt angle reduces below the threshold, pumping may resume automatically. By sensing the movement or title angle during a pumping session, the system may also derive the user's activity such as walking, standing or lying. The accelerometer determines if the liquid is sufficiently still to accurately measure or infer the quantity of the liquid in the container. Thus, unreliable fill level measurements will be discharged.

The above proposal proves the attempts to provide means to accurately determine the fill level within the milk container. Determination of the fill level of the milk container of an in-bra breast pump is an ambitious task as the milk container is constantly moving. Moreover, the spatial relationship between the surface of the milk within the container and any sensor cannot be assumed to be known like in a breast pump with a milk container resting on a surface or being designed such that the milk container of the breast pump can with good confidentiality be assumed to be hanging below the breast shield with the user of the breast pump sitting in an upright position and holding the breast pump in such position.

The present invention aims to propose a new concept of assessing yield of the breast pump during a pumping session and/or a new concept of assessing the fill level within the milk reservoir of the breast pump.

As a solution to the above object, the present invention provides a breast pump wearable inside a bra. In other words, the entire breast pump has a size and shape, which is adapted to be worn inside a bra. The outer surface, which projects the breast of the user in a use condition of the breast pump usually imitates the shape of a female breast. The breast pump has a breast shield adapted to at least in part receive a breast of a lactating user. The breast shield usually has a nipple tunnel which receives the nipple of the breast and may provide a path for the expressed milk to be further advanced towards the milk container. The wearable breast pump has an aggregate for generating suction for expressing the milk from the breast. Said aggregate is usually energized by a rechargeable battery within a housing of the breast pump. The aggregate may be any suitable means for generating a negative pressure adapted to express milk from the breast. The aggregate may be a motor driving a reciprocating suction generating means, which reciprocating suction generating means forces a fluid within the breast pump to generate a negative pressure within the breast shield for expressing the milk. Such a reciprocating suction generating means may in general comprise or be formed of a piston, a diaphragm driven in a reciprocating fashion by mechanical advancing means and/or by a fluid (liquid or gas). Such a reciprocating suction generating means may comprise or be formed of piezo means of a piezo air pump and/or a membrane of such piezo air pump or drive means or a drive element of a single or multi-stroke air pump or a diaphragm of such a multi-stroke air pump. The reciprocating suction generating means may comprise or be formed of a stiff or a flexible moving member, which member by a single or multiple strokes is suitable to generate a full vacuum, i.e. the maximum (in absolute value) obtainable suction pressure. The reciprocating suction generating means may be contained within the housing and may cooperate with a membrane, which membrane defines a pumping chamber within the breast pump. Alternatively, the aggregate may be formed by a component of an air pump or such pump in total, which a component or air pump in total generates varying air pressure levels as known from WO 2018/229504 A1.

The following description will refer to specific conditions of the reciprocating suction generating means by referring to the piston, which referral is made to improve the understanding the feature/thespecific condition but shall not be understood to limit the term to apply only to the embodiment of the pump with a piston. In particular, the latched piston position, the latched piston and/or the piston position mentioned below shall likewise apply to embodiments with reciprocating suction means other than a piston.

The breast pump furthermore has a milk container providing a reservoir for the expressed milk. This reservoir communicates with the breast shield, specifically the nipple tunnel of the breast shield and usually has a venting hole or spout opening to the outside environment. One or plural valves may be provided between the nipple tunnel and the milk container to direct the flow of milk and possibly allow a certain degree of reflux as e.g. known from WO 2019/080995A1.

The breast pump furthermore has a controller which is operatively connected with the aggregate for controlling the same. The controller usually is operatively connected with a user interface on the outer side of the breast pump, in particular on the outer side of a housing, which housing receives the aggregate, a power source in form of a battery and other, in particular electric, components.

The present invention is preferably based on the concept to make the breast shield and the milk container of a material that can be cleaned in a dish washer and to configure the breast shield and the milk container in a way that the same can be connected with each other and/or the housing to form the unitary breast pump. The breast shield and the milk container can releasably be attached to each other and/or the housing. The breast shield and/or the milk container may be formed of one or plural components. In particular, the milk container may be adapted to be used as a storage for storing the amount of expressed milk.

According to the present invention, the aggregate comprises reciprocating suction generating means, in particular a piston, which reciprocating suction generating means are moved in a reciprocating manner for generating a specific pressure profile acting on the breast received within the breast shield. Further volume flow assessment means are provided to estimate the volume flow on the basis of the movement of the reciprocating suction generating means. In general, the movement of the reciprocating suction generating means in this preferred embodiment is assigned to a respective yield of expressed milk. Ideally, the stroke volume of the reciprocating suction generating means, in particular the piston is assumed to correspond with the volume of expressed milk per stroke.

This assumption may be suitable to assess the volume flow within the pump. However, the accuracy of the calculation of the milk content based on the movement of the reciprocating suction generating means may be improved by taking into account the following phenomena:
Due to friction and/or leakage, a certain movement of the reciprocating suction generating means may not correlate with or result in milk flow. This fact will be taken into account by a latched piston position, which latched piston position corresponds to a position of the reciprocating suction generating means, in particular the piston in which the pump is intended to provide milk flow but will not do so as e.g. the negative pressure is lowered by leakage within the system allowing air to enter the system, in particular in the milk path between the nipple tunnel and the reservoir. The latched piston position is indicative of a position of the reciprocating suction generating means, which is intended to generate suction but cannot be correlated with milk flow from the breast or within the breast pump and/or does not result in the expression of milk from the breast as e.g. measured within the pump, and thus does not lead to any milk flow within the breast pump due to the activation of the aggregate. The latched piston position may be stored in the latched piston position memory as a pre-set value. This value may be set on the basis of the overall leakage which generally occurs when operating the breast pump. The overall volume flow is assessed by said volume flow assessment means by decreasing the stroke of the reciprocating suction generating means by the latched piston position. In other words, only the portion of the stroke which can be assigned to milk flow will be taken into account when estimating the milk flow based on the movement of the reciprocating suction generating means.

The latched piston position may e.g. be measured in a stimulation phase, which stimulation phase is an initial phase of a pumping session for expressing milk and which stimulation phase is followed by an expression phase in which milk is expressed from the breast. In other words, the initial operation of the breast pump in each pumping session can be assumed to be a no milk flow condition, in which phenomena observed within the pump are not related to flow of milk.

According to a preferred embodiment, a sensor is provided for determining the milk level within the reservoir. For example as the sensor, an infrared sensor is provided, usually attached to and/or received within the housing and assigned to the reservoir for determining the milk level within the reservoir. The preferred infrared sensor can be any sensor emitting and receiving electromagnetic waves. The infrared sensor may be based on the principle of transmitting the electromagnetic waves to at least a portion of the milk container or by reflecting the electromagnetic waves, in particular on the surface of the milk within the milk container for determining the milk level with the reservoir of the milk container. Other examples of sensors are e.g. inductive sensors. In fact, any sensor can be used which is suitable to determine the fill level within the reservoir.

The controller may be adapted to output a signal indicative of the fill level within the reservoir. Combined processing of signals from the sensor and the volume flow assessment means will improve accuracy in the assessment of the fill level within the reservoir.

A more accurate assessment of the fill level within the reservoir may take into account the inclination of the milk container within the field of gravity. For this, the controller may be operatively connected with an accelerometer. This accelerometer senses the orientation of the breast pump in the field of gravity and thus is adapted to provide a signal indicative of the inclination of the breast pump, namely the milk container within the field of gravity. The accelerometer can measure the tilt angles in the front direction and in the transverse direction with reference to the vertical defined by the force of gravity. The accelerometer may be adapted to detect accelerations in all three directions within a cartesian coordinate system. Processing of the signals may allow indication of a moving distance and/or change of position within the room. The signals of the accelerometer will be taken into account when processing said signal of the sensor.

The controller may have a look-up table to correct a specific signal received from the sensor on the basis of the signal received from the volume flow assessment means and/or the accelerometer of the pump. The look-up table may be stored within a memory of the controller and may be based on experimental results correlating the exact volume of milk within the reservoir with the signals of the sensor depending on the inclination of the milk container sensed by the accelerometer and/or volume flow obtained as a result of a specific movement of the reciprocating suction generating means.

The accelerometer will usually be adapted to provide information on the inclination of the breast pump in any direction. Also other ways to link the signals from the sensors and accelerometer are possible, for example analytical equations or relationships calculated on the basis of the breast pump design or experimental data.

To improve accuracy, the controller may have a data storage with historic data, which historic data may result from actual measurements of the volume flow assessment means and/or the sensor of the specific breast pump having the data storage or may be have been stored in the data storage on the basis of general calculations or simulations or by analysing historical or operational data of multiple other pumps. The historic data preferably is data of the same pumping session as the pumping session when yield of the pump is assessed. For example, the controller can be adapted to be connected by cable or wirelessly to the internet and/or cloud.

As described above, the present invention provides means for improving the accuracy of the signal released by the controller.

The solution of WO 2018/229504 A1 has a single infrared sensor calculating the volume within the reservoir on the basis of the time elapsed between releasing the electromagnetic wave from the infrared sensor and receiving the signal reflected by the surface of the milk within the reservoir. Such solution will provide a signal indicative of the milk level within the reservoir which is greatly affected by the inclination of the breast pump. The present invention may employ a single sensor, the signal thereof being processed in the controller. In this preferred embodiment, the controller is adapted to access and output a signal indicated of the yield of the breast pump and/or the milk level within the reservoir on the basis of both, the volume flow assessment means and the sensed milk level sensed by the sensor.

In order to further improve accuracy, the present invention proposes plural sensors, in particular infrared sensors, each of which being assigned to the reservoir. The sensors are spaced apart from each other to provide a signal indicative of a specific fluid level within the reservoir. The plural infrared sensors emit electromagnetic waves to different locations of the reservoir and thus observe the milk level at different locations within the reservoir. Those different locations may in particular be assigned to different fill levels in height direction of the reservoir in an assumed orientation of the milk container. In the following description, this assumed orientation may be regarded to correspond with an orientation in which a nipple tunnel of the breast shield extends essentially in horizontal direction while the breast pump is in an upright position, in which the milk container can be assumed to be provided with the bottom of the milk container defining the lowermost portion of the milk container in the field of gravity. The plural sensors are usually provided to give information on different fill levels. The different sensors are usually spaced apart from each other vertically in the assumed orientation.

In case of an infrared sensor arrangement, as the milk volume builds up within the reservoir, a lowermost infrared sensor is the first to give a signal indicative of the presence of milk at the position of this first infrared sensor. As the volume of milk within the reservoir builds up further, a second infrared sensor which is arranged higher than the first infrared sensor, will give a second signal providing information that the milk level has reached this second milk level. However, even under ideal conditions, in which the breast pump is held completely stationary with a milk level constantly building up with no milk slushing in the reservoir due to movement of the user of the pump, the controller does not have any information of the fill level between the first and the second sensor until the milk level reaches the second infrared sensor or after the milk level has overcome the second sensor.

For this problem, the controller preferably is adapted to assess and output a signal indicative of an intermediate milk level between two specific milk levels based on information from the flow assessment means. Thus, an assessed flow or yield is calculated by the controller to contribute to additional volume of milk within the reservoir. This additional volume of milk is added to the volume sensed by the lower infrared sensor. As the volume flow is taken into account when calculating the overall volume of milk within the reservoir, the signal of the controller indicative of the overall volume within the milk container does more accurately correspond to the actual milk volume within the reservoir. Thanks to this measure, intrusiveness of prevention or counteract or alert actions indicating a full milk container or a risk of milk spillage are further reduced, because milk amount within the reservoir is better known and larger tilt angles for the breast pumps may be allowed without triggering an alarm.

To further improve the above concept, the present invention proposes a pressure sensor adapted to generate a suction pressure signal indicative of a suction pressure generated by the aggregate. The controller is operatively connected with said pressure sensor to determine the latched piston position based on the signal of said pressure sensor. In other words, the reciprocating suction generating means, in particular the piston is arranged in a position within a stroke, which position usually is intended to generate a negative pressure but which position does not result in any suction due to friction or leakage or the like. Thus, the pressure sensor may not provide a signal indicators of a negative pressure. Accordingly, the pressure sensor in addition with a signal indicative of the position of the reciprocating suction generating means, in particular the piston of the pump may be used to sense and thus determine the latched piston position and thereby assess the volume flow based on the effective movement of the reciprocating suction generating means, in particular the piston in which movement suction is generated and thus milk is assumed to be expressed. The latched piston position identified on the basis of the pressure sensor may be stored in the latched piston position memory to utilize the latched position for plural strokes without need to evaluate the signal of the pressure sensor for each stroke. Such operation may e.g. be feasible under the assumption that after assembly of the pump, the factors preventing yield milk flow during operation of the pump do not change. Under such assumption, a leak volume for each stroke may be assumed to be constant. Thus, correction of the volume flow based on the movement of the pump may be carried out for each stroke by only one single pressure sensor measurement for determined the latched piston position for a single pumping session.

The pressure sensor may e.g. observe the reduction of the suction pressure within the breast pump as the reciprocating suction generating means have been moved to maximally expand the volume of the pumping chamber which volume is cyclically changed by the movement of the reciprocating suction generating means to generate suction for expressing milk. In such a position of the reciprocating suction generating means and a no flow condition in which no milk flows within the breast pump, reduction of the suction pressure is assigned to a leak within the system. The leak volume corresponds with the volume assignable to the latched piston position. The no flow condition may e.g. be assumed in a stimulation phase in which the aggregate is operated in a different mode than in the expression phase, which expression phase is followed by the stimulation phase in a pumping section. In the stimulation phase, no milk is expressed while a negative pressure may be generated by the aggregate.

The signal of the pressure sensor mentioned above may be assigned to an operational parameter of the aggregate, which operational parameter is a set point of the control. However, as this set point may not reflect the actual position of the reciprocating suction generating means, a preferred embodiment of the present invention has a piston position sensor which is adapted to generate a signal indicative of a position of the reciprocating suction generating means. The controller is operatively connected with said piston position sensor to determine the latched piston position based on said position signal. Accordingly, the true position of the reciprocating suction generating means is relied upon when determining the latched piston position on the basis of the suction pressure signal of the pressure sensor. The piston position sensor may be an encoder which is assigned to a component within the drive train, e.g. from a motor to drive the reciprocating suction generating means, including the shaft of an electric motor driving the reciprocating suction generating means, in particular the piston or a nut element threadably engaging the reciprocating suction generating means, in particular the piston for advancing the reciprocating suction generating means, in particular the piston in a linear movement upon rotation of the nut element. The piston position sensor may as well monitor axial displacement of the reciprocating suction generating means, in particular the piston or another component attached to or cooperating with the reciprocating suction generating means.

The above measures are proposed to improve volume flow assessment on the basis of the movement of the reciprocating suction generating means, in particular the piston.

According to an alternate proposal, which may also be utilized in combination with the above assessment of the volume flow on the basis of the movement of the reciprocating suction generating means, the breast pump according to the invention may comprise a pressure sensor adapted to generate a suction pressure signal indicative of a suction pressure generated by the aggregate. In this embodiment, the controller is operatively connected with the pressure sensor. The volume flow assessment means are adapted to estimate the volume flow on the basis of the at least one operational parameter of the aggregate and the suction pressure signal. The suction pressure signal may be assigned to a specific volume flow. Thus, the controller may have a look-up table, in which a specific negative pressure within the breast pump measured by the pressure sensor is assigned to a specific volume flow. Such memory may for example assign a specific volume of milk to an overall suction profile measured for each stroke of a membrane generating the suction pressure on the milk side of the breast pump.

The controller may have a look-up memory, which assigns sensed pressure profiles of a single pumping cycle with a volume flow within the pump. The memory may be provided with information stored on the suction level during a single pumping cycle at no flow. Any deviation from said profile sensed by the pressure sensor may be assigned to milk flow within the pump. The memory will as well have different pressure profiles stored therein which are each assigned to a specific volume flow. By comparing the sensed pressure profile with the recorded profiles, the controller may select an appropriate value for the milk flow within the pump upon the sensed pressure information.

According to a preferred embodiment of the solution having a pressure sensor for assessing the volume flow within the pump, the aggregate comprises a reciprocating suction generating means, in particular a piston, which reciprocating suction generating means are moved in a reciprocating manner for generating the suction. The volume flow assessment means are adapted to estimate the volume flow on the basis of a suction pressure difference sensed by pressure sensor as the reciprocating suction generating means, in particular the piston is held stationary in a suction-generating position. This suction-generating position usually is the position in which the reciprocating suction generating means generate the maximum pressure, i.e. is the position corresponding to the maximum stroke position of the reciprocating suction generating means, in particular the piston. The reduction of the negative pressure as the reciprocating suction generating means are held stationary, can be assigned to the volume entering the milk path as a consequence of the constant negative pressure. In other words, the pressure difference in the milk path can be assigned to the milk volume flow. Accordingly, the embodiment usually has a look-up table which the negative pressure signal of the pressure sensor will be assigned to a specific yield of volume of expressed milk by said reciprocating suction generating means in the stationary position. This preferred embodiment is in particular based on the concept of holding the reciprocating suction generating means for a longer time in the maximum stroke position to express milk from the breast at maximum negative pressure. In other words, the embodiment is in particular based on the concept of having an expansion phase in which a pumping chamber on the milk side is expanded to increase the negative pressure on the milk side, and a holding phase, in which the reciprocating suction generating means are held for a specific period of time at a maximum expanded reciprocating suction generating means, in particular at a maximum expanded piston, and a reduction phase in which the reciprocating suction generating means, in particular the piston are/is moved to reduce the volume in the pumping chamber to first reduce the level of negative pressure within the milk path and at the end transfer the milk from the milk path within the milk pump into the reservoir.

Before a new expansion phase starts, the reciprocating suction generating means may be stationary in a relax phase, which relax phase is an intermediate phase between the end of the reduction phase and the beginning of the next expansion phase. When transferring the milk to the reservoir, the pressure within the nipple tunnel may still be negative due to a base line valve provided between the nipple tunnel and the pumping chamber, cf WO2019/080995 A1.

The accuracy of the volume flow assessment based on the signal of the pressure sensor may be improved by a latched memory adapted to store a latched pressure, which latched pressure is indicative of a pressure difference sensed by the pressure sensor at no milk flow condition. At a no milk flow condition and preferably during the maximum stroke rest phase, any change in the negative pressure value can be assigned to leakage flow. The volume entering the milk path by leakage corresponds to the volume which does not contribute to milk flow into the reservoir. Thus, measuring or at least assessing the portion of the measured pressure sensor assigned to other phenomena than milk flow within the pump will improve accuracy of the volume flow assessment.

The latched pressure may e.g. be measured in a stimulation phase, which stimulation phase is an initial phase of a pumping session for expressing milk and which stimulation phase is followed by an expression phase in which milk is expressed from the breast. In other words, the initial operation of the breast pump in each pumping session can be assumed to be a no milk flow condition and may e.g. be used for an initial determination of a latched pressure. The latched pressure may correspond to a difference in the negative pressure as the reciprocating suction generating means are held stationary in a suction-generating position, in particular in the maximum stroke position. With no movement of the reciprocating suction generating means, in particular the piston, any difference in the pressure signal can be assigned to leakage.

As mentioned above, the stimulation phase per definition within the logic of the controller is a phase in which a no flow condition exists. However, it may be advantageous to determine a latched position of the reciprocating suction generating means throughout a pumping section and thereby improve reliability of the volume flow assessment on the basis of an operational parameter of the aggregate. For this, the controller preferably is adapted to determine a no milk flow condition during an expression phase. Such adaption of the controller may be implemented by an analysis within the controller, which analysis will compare a sequence of sensor signals to arrive at a conclusion that a no flow condition exists. For this, a sequence of sensor signals will be analyzed, which signals may be the signal of the pressure sensor or the infrared sensor to conclude from the signals that no milk flow has been transferred to the reservoir. The controller may have a memory in which typical sensor signals and, in particular, a sequence of sensor signals is stored, which signals indicate no flow into the reservoir. Such data may have e.g. been stored in the memory based on experimental results on a typical behaviour of a respective breast pump at no flow conditions.

To further improve accuracy, the controller is adapted to periodically determine a no milk flow condition during one pumping session. Thus, the latched pressure or latched piston position is periodically assessed. The new value for the latched pressure or latched piston position may be entered into the register while overwriting the old value. In a pumping session and though the expression phase may have been already activated, the breast of a user will for some period of time not release any milk. Those intervals will in particular be suitable to periodically determine a no flow condition.

According to the concept of the present invention, the information obtained with the improved means and methods are entered into the controller of the aggregate, e.g. to stop the aggregate, if the milk collected in the reservoir has reached the maximum volume. As known from WO 2018/229504 A1, the controller may at least temporarily stop the aggregate in case of excessive movement of the user noticed by the accelerometer. Moreover, the controller may be operatively connected with a user interface to output information indicative of the milk level within the reservoir. Thus, the means provided to more accurately determine the volume of expressed milk within the reservoir is likewise used to indicate the yield of expressed milk to the user during a pumping session.

Further preferred embodiments of the present invention will become apparent from the following description of an embodiment in combination with the Figures.

The present invention will now be described by referring to figures showing some examples. In the figures
- Fig. 1: is an exploded view of the individual components and elements of a breast pump having a milk container having transparent sections;
- Fig. 2: is a sectional view of the breast pump in an assembled state;
- Fig. 3: is a perspective top view on an inner surface of the housing;
- Fig. 4: views of a schematic functional model of a pumping chamber with piston of the embodiment of Figures 1 and 2;
- Fig. 5: pressure profiles during a pumping cycle, and
- Fig. 6-11: shows the breast pump with different tilt angles.

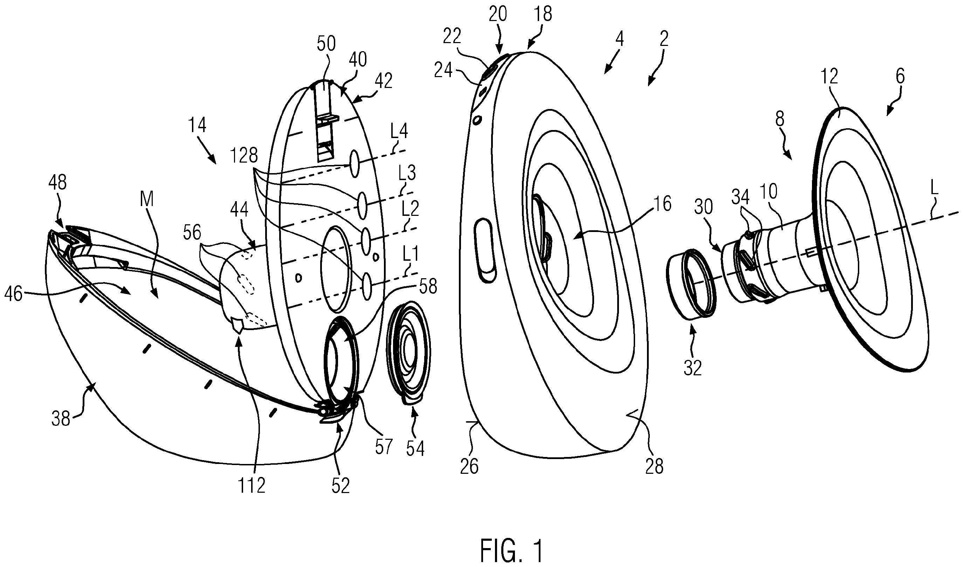

Fig. 1 shows the breast pump 2 shaped at least in part to fit inside a bra and comprising the housing 4, a breast shield 6 provided element 8 with a nipple tunnel 10 adapted to receive a nipple and a flange 12. The breast pump 2 has a milk container 14 adapted to contain a predetermined amount of expressed milk M.

The housing 4 is ring-shaped and circumferentially encloses a bore 16. L identifies a longitudinal axis L extending through the bore 16. On the circumference of a top section 18 of the housing 4 a user interface 20 is provided having buttons 22 and optical indicators 24 of indicating operational information of the pump. As derivable from Fig. 1, a front surface 26 of the housing 4 adjacent to the milk container 14 is flat whereas a rear surface 28 of the housing 4 is concave and imitates the shape of a female breast.

The breast shield element 8 is made of a transparent material. Next to a free inner end 30 of the nipple tunnel 10, Fig. 1 shows a two-way valve element 32 made of flexible material which two-way valve element 32 can be mounted on the free inner end 30 of the nipple tunnel 10.

The breast shield element 8 is provided with threads 34 arranged circumferentially spaced relative to each other on the outer circumference of the nipple tunnel 10.

The milk container 14 consists of a spherical container shell element 38 and a lid element 40. This lid element 40 comprises a flat lid section 42 and a longish nipple tunnel receptacle 44. Reference numeral 46 identifies a reservoir sealed between the inner wall of the spherical container shell element 38 and the flat lid section 42 in the assembled state of the milk container 14. Into said reservoir 46, the nipple tunnel receptacle 44 extends.

A top section of the spherical container shell element 38 is provided with a spout 48. The flat lid section 42 slidingly holds a spout closure element 50, which can be slid to open and close the spout 48. A hinge 52 releasably connects the container shell element 38 and the lid element 40.

The inner circumference of the nipple tunnel receptacle 44 is provided with threads 56 cooperating with the threads 34 of the nipple tunnel 10 to secure the breast shield element 8 against the milk container 14 when the nipple tunnel 10 projects through the bore 10 of the housing 4 and is received within the nipple tunnel receptacle 44.

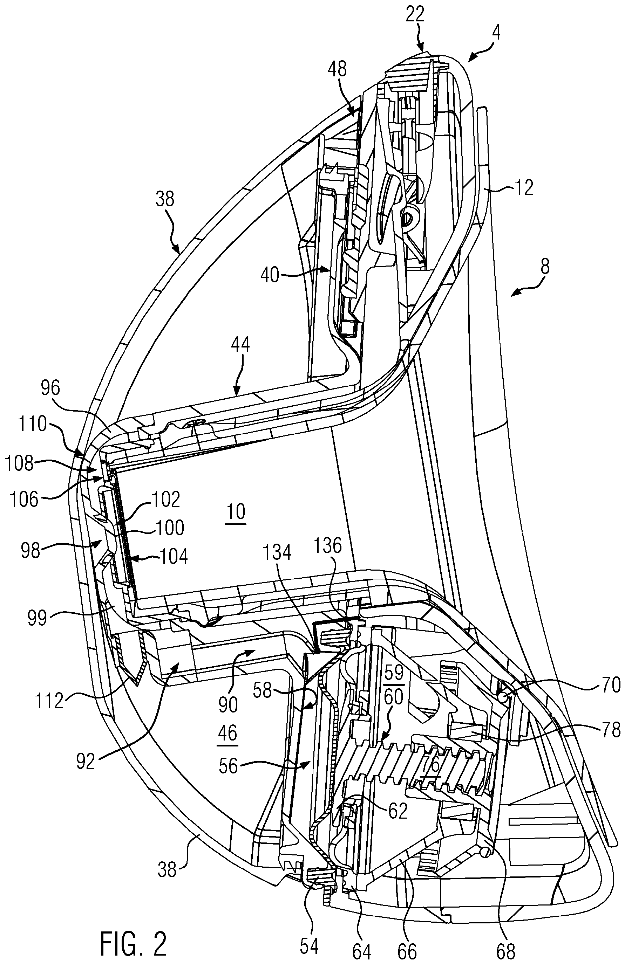

Reference numeral 54 identifies a membrane, which is sandwiched between the milk container 14 and the housing 4 for closing a membrane working chamber 57 formed by a trough 58 projecting from the flat lid section 42 toward the reservoir 46, see Fig. 2.

As can be seen from Figure 2, this membrane 54 is in contact with a reciprocating suction generating means 59 in form of a piston 60 having a cap section 62 sealingly coupled with an aggregate membrane 64, which is sandwiched between the housing 4 and a drive housing 66 received within the housing 4. The drive housing 66 rotatably supports a nut element 68. The nut element 68 cooperates with a drive belt 70 to drivingly connect the nut element 68 with a drive shaft 72 of an electric aggregate 74. The nut element 68 cooperates with a spindle 76 of the piston 60. The nut element 68 is rotatably supported by a roller bearing 78 held by the drive housing 70.

Reference numeral 80 identifies a rechargeable battery/accumulator adapted to energize the electric aggregate 74 and a controller 82. For charging, the rechargeable battery 80 is coupled with a USB interface on the outer side of the housing.

Fig. 2 elucidates details of a pumping chamber 90 including a membrane working chamber 57 provided between the membrane 54 and the trough 58, a pumping channel 92, an outlet space 94 provided between the free inner end 30 of the nipple tunnel 10 and a forward closed end 96 of the nipple tunnel receptacle 44. This closed end 96 has a projection 98 defining a flat closing surface 100 cooperating with the two-way valve element 32. The center of the two-way valve element 32 has a reflux opening 102, which in cooperation with the flat closing surface 100 defines a reflux valve 104. In Fig. 2, reference numeral 106 identifies a milk outlet opening the two-way valve element 32, which is provided by the breast shield element 38. This wall section 108 in combination with the milk outlet opening 106 defines a milk outlet valve 110.

As derivable from Fig. 2, the lower end of the outlet space 94 is provided with a container valve 112 sealing the pumping chamber 90 against the reservoir 46. This container valve 112 prevents reflux of milk contained within the reservoir 46 into the outlet space 94. The container valve 112 is formed by a separate element formed made of a soft elastomeric material and attached to the milk container 14, specifically, the nipple tunnel receptacle 44. The container valve 112 may be affixed to the lid element 40 or may be separatable from the lid element 40 for cleaning or replacement.

For operation, the nipple will be arranged within the nipple tunnel 10. Next, the pump 2 via a button 22 of the user interface 20 is activated. Starting from an initial position, in which the piston 60 projects toward the milk container and thus projects into the membrane working chamber 57 as shown in figure 2, the piston 60 will be moved to the right side of figure 2. Thus and during this expansion phase, a negative pressure is built up in the pumping chamber 92. By way of example, the piston 60 may be brought into the maximum stroke position, in which the piston is retracted into the housing and with maximum displacement. Arrival of the piston in the maximum stroke position is the end of the expansion phase. In this maximum stroke position the movement of the piston 60 will pause. After holding the piston 60 stationary in the maximum stroke position (holding phase), a reduction phase is initiated, in which the piston 60 will be moved towards the milk container 14 and into the membrane working chamber 57. The piston 60 will reach the initial position and will rest in this initial position for some time (relax phase).

As the reflux valve 104 is closed, the negative pressure will build up in the nipple tunnel 10 through the open milk outlet valve 108. Extracted milk is drawn through the milk outlet opening 106 in a reduction cycle of the suction source 6. In a reduction phase, in which the aggregate membrane 84 and thus the protective membrane element 60 is moved towards the membrane working chamber 57, the milk outlet valve 118 will close. The suction pressure within the pumping chamber 92 will be reduced, i.e. the pressure will rise. The reflux valve 104 will remain open until a reduced threshold suction pressure is reached within the nipple tunnel 10. Thus, a negative pressure will be maintained as a minimum or baseline suction pressure within the nipple tunnel 10, cf. WO2019/080995A1. At appropriate pressure difference, milk M will flow through the container valve 122, which is a one-way valve, into the reservoir 46. In an expansion phase, in which the aggregate membrane 84 and thus the protective membrane element 60 is moved towards the drive housing 70, the suction force within the pumping chamber 94 is increased. Eventually, the container valve 112 as a one-way valve will close to prevent reflux of milk M from the reservoir 46 into the pumping chamber 92. The milk outlet valve 110 will open to allow milk to flow from the nipple tunnel 10 into the outlet space 94 of the pumping chamber 90, which pumping chamber 90 is essentially completely filled with milk M. Thus, the level of extracted milk M within the reservoir 46 builds up during operation of the pump while the nipple tunnel 10 is filled with milk M and the nipple is constantly provided within a negative pressure environment.

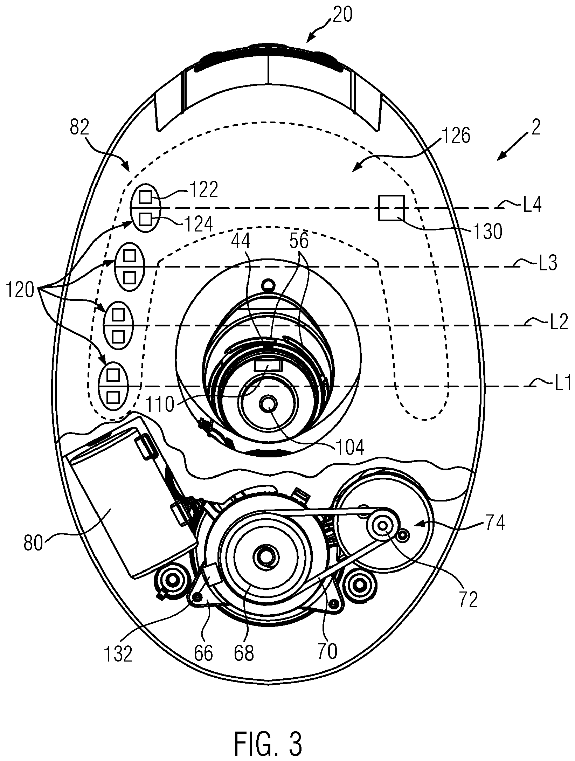

As can be seen in Fig. 1 and 3, the breast pump 2 further comprises plurality of infrared sensors 120 each including an emitter 122 for an emission of electromagnetic waves and a receiver 124 for a reception of the electromagnetic waves, each infrared sensors 120 is designed to provide a sensor signal for a detection of expressed milk M. The electromagnetic waves are in the form of infrared light. The controller 82 has a printed circuit board 126 for controlling the suction source 6 and adapted to receive signals of the infrared sensors 120. The infrared sensors 120 are mounted on the printed circuit board 126. The lid element 40 has windows 128, which each are at least partially transparent to the infrared waves, which are arranged to be in contact with the expressed milk M and which are each assigned to one of the infrared sensors 120.

The windows 128 as well as the infrared sensors 120 are assigned to four fill levels L1, L2, L3, L4. The highest fill level L1, L2, L3, L4 which is the last fill level L4 pertains to the amount of 120 ml milk in the milk container 14 when the pump 1 is arranged in an assumed position. In the assumed position the breast pump 2 upright i.e. the axis L extends horizontally and the spout 48 assumes the highest point of the milk container 14 in the field of gravity.

The controller 82 is connected to a further sensor in form of an accelerometer 130 of the breast pump 2, which accelerometer 130 is mounted on the printed circuit board 126. The accelerometer 130 can be any sensor providing a signal indicative of the orientation of the breast pump 2 in the field of gravity. Reference number 132 identifies a piston position sensor in form of an encoder assigned to the nut element 72. The piston position sensor 132 via the angular movement of the nut element 72 is used to determine the position of the piston 60. Reference number 134 identifies a pressure sensor, which is received within the material forming the milk container 14 by integrally molding. The pressure sensor 134 is operatively coupled with the controller 82 via contact pads 136 at an interface between the milk container 14 and the housing 4.

The controller 82 is adapted to receive and process the sensor signals of all of the sensors 120, 130, 132, 134. In this regard, the printed circuit board 126 may hold mounted a CPU, GPU, RAM, a memory, and in particular holds the sensor 120 and 130.

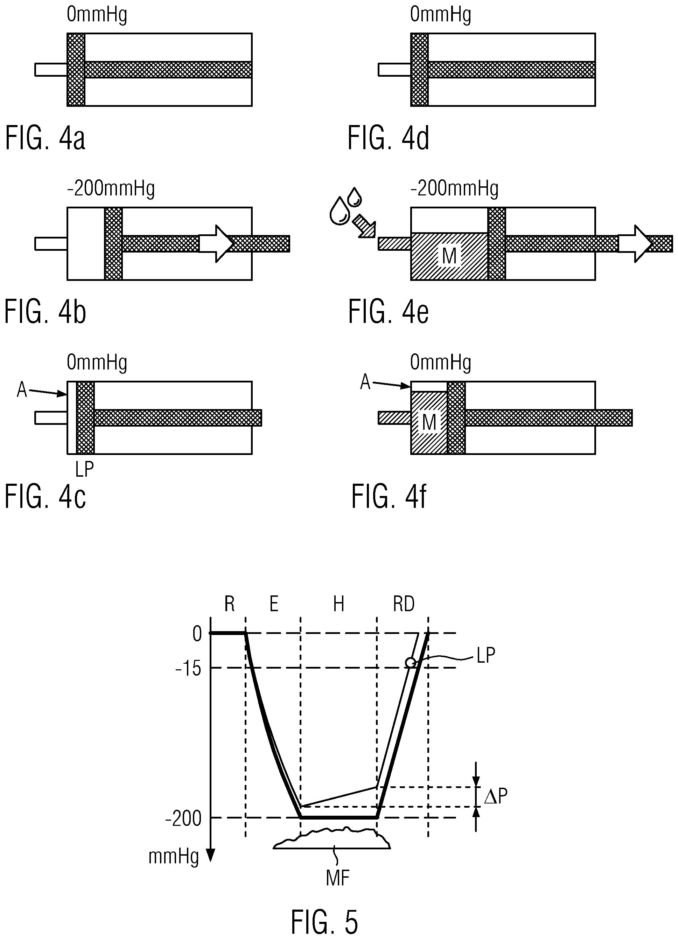

For the following description of different means to assess the volume flow into the reservoir 46, reference is made to Fig. 4 providing the basis for an appropriate understanding of the latched piston position.

Fig. 4 simplifies the pumping chamber 90 and the piston 60 which is assumed to correspond to a piston within a cylinder. The left-hand side of Fig. 3 shows the situation without a milk flow. At Fig. 4a, the reduction phase has been completed. The pumping chamber 90 assumes a minimum volume. As the piston 60 is retracted and moved to the right-hand side of Fig. 4, a suction pressure builds up. As the nipple tunnel 10 is sealed with no milk being expressed from the breast, no flow is generated and the negative pressure built up within the pumping chamber 90 corresponds to the displacement of the piston 60.

When reducing the volume of the pumping chamber 90 in the movement from b to c, the suction pressure within the pumping chamber 90 becomes higher. The negative pressure assumes lower absolute values.

The left-hand side of Fig. 4 assumes leakage of air within the system. In other words, the suction during the expansion and reduction cycle of the piston 60 led to some degree of fluid flow into the pumping chamber 90. Accordingly, a 0 mm Hg position of the piston 60 after one cycle does in Fig. 4c not correspond with the initial position shown in Fig. 4a. The remaining volume within the cylinder is assumed to be filled with air A, which air A entered to the system due to leakage.

The volume corresponding to the diameter of the cylinder and a latched piston position identified with LP in Fig. 4c corresponds with the latched volume, which latched volume is volume which was not used for drawing milk into the pumping chamber 90 and pushing this milk into the reservoir 46 during one pumping cycle.

The effect of latched position LP in case of milk flow is shown in Figs. 4d through f. After one cycle and at a pressure of 0 mm Hg, a residual volume of milk M is contained within the cylinder. The amount of air A trapped in the cylinder corresponds with the amount of air at the latched position LP in Fig. 4c.

The above discussed phenomenon will be taken into account in the described embodiment when assessing the volume flow within the breast pump 2.

From the above description, it is evident that each of the infrared sensors 120 can only provide information on a specific milk level within the reservoir corresponding to the levels L1 through L4 in case the breast pump 2 is provided in an assumed orientation with no movement of the breast pump 2 at all. An intermediate milk level between each of the levels L1, L2, L3, L4, respectively, may be calculated by the controller 82 based on information of the volume flow. According to a first embodiment, positional information of the piston 60 is used for assessing the volume flow.

With some accuracy, it can be assumed that the volume of milk pushed into the reservoir 46 in each cycle corresponds with the volume which is reduced in each reduction phase or generated in each expansion phase. Thus, each stroke of the piston 60 provides some information on the volume flow. As the movement of the piston 60 is an operational parameter known by the logic of the controller 82, the controller 82 may calculate the volume flow on the basis of the known geometrical constitution of the piston 60 and the volume change in each expansion or reduction phase.

Due to possible leakage of air into the pumping chamber, a memory of the controller 82 is adapted to store a latched piston position LP. This latched position may e.g. be obtained by utilizing the signals of the pressure sensor 134. During a stimulation phase, in which the controller 82 assumes a no flow condition, the controller 82 may compare two positions of the piston 60, one position being a position before an expansion phase E and the other position being a position after a reduction phase RD, in which positions an identical pressure is measured. The positional difference between the first and the second position identified by the piston position sensor 132 corresponds with the volume of air entering the pumping chamber 90 due to leakage. Respective value may be entered into a memory of the controller 82 as the latched piston position LP.

This spindle position method for assessing the flow volume will be described by referring to Fig. 5, which is a graph on the negative pressure in case of no leak building up in the pumping chamber 90 in one pumping cycle. During a rest phase R, the piston 60 is stationary. In the expansion phase E, the volume of the pumping chamber 90 is expanded. Suction pressure builds up. The lower full curve identifies the suction pressure being built up in absence of any milk flow. After reaching a maximum stroke position corresponding to -200 mm Hg, a holding phase H is initiated. In absence of any flow, the suction pressure remains constant. After said holding phase H, the reduction phase RD is initiated. The negative pressure assumes higher values. At the end of the stroke, the relax phase R is reached at 0 mm Hg.

The upper curve elucidates the situation with milk flow MF. In the expansion phase E, milk already flows into the pumping chamber 90. During the holding phase H, the negative pressure leads to further milk entering into the pumping chamber 90. Thus, the suction pressure is not constant during the holding phase H. In fact, the suction force is lower at the end of the holding phase H. In the reduction phase RD, the negative pressure assumes higher values, the milk flow MF is lowered. Finally, milk is pushed out of the pumping chamber 90 and is transferred into the reservoir 46. Fig. 5 is idealized. In fact, with a flow of milk, the 0 mm Hg level is reached earlier with the consequence that a positive pressure is built up in the last portion of the reduction phase RD, which positive pressure will push the milk into the reservoir 46.

At -15 mm Hg, the latched spindle position LP is reached. In other words, the remaining stroke will essentially be used to advance air drawn into the pumping chamber 90 out of the pumping chamber and into the reservoir 46, which air is eventually vented through the spout 38.

The position in accordance with Fig. 4c for the spindle position method corresponding to the latched piston position will be reduced from the stroke of the piston stored either in the memory of the controller 82 or identified by the signals of the piston position sensor 132 for each stroke. The volume of milk advanced into the reservoir will be calculated on the basis of the effective stroke, i.e. the absolute stroke minus the latched piston position.

As shown, the signal of the pressure sensor 134 can be utilized to determine the latched piston position LP and thus to improve the accuracy of the assumption that the stroke volume of the piston 60 corresponds with the volume of milk flow during one piston cycle.

Fig. 5 can likewise be used to explain the alternate concept to identify a latched pressure. In case of a no flow condition, a rise in measured pressure difference during the holding phase H, which pressure difference is identified with Δp in Fig. 5 can be assumed to correspond with the loss of the suction pressure due to air leaking into the pumping chamber 90. This latched pressure Δp can be determined at a no flow condition in which any sensed pressure difference during the holding phase H can be assigned to leakage of air. Respective latched pressure Δp may be entered into a memory. The holding phase H may be identified by the controller 82 on the basis of operational instructions of the controller 82 to move the piston 60 and thus activate the aggregate 74 or to pause the aggregate 74 in the holding phase H.

As slip may occur between the drive shaft 72 and the drive belt 70, a more accurate way is to actually determine the exact position of the piston 60 by the piston position sensor 132 and assign the signal of the piston position sensor 132 to the holding phase H and/or the exact position of the piston 60 and/or positional changes as the suction pressure is measured by the pressure sensor 134.

While determination of the latched piston position LP and/or the latched pressure Δp was described for a case in which the piston 60 is stationary, the amount of leakage may likewise be obtained as the aggregate 74 is operated. Processing of the signals of the piston position sensor 132 and the pressure sensor 134 may be more complex but feasible. In this respect, it is worth noting that the encoder providing the piston position sensor 132 and/or the pressure sensor 134 may sample signals at a rate of between 200 and 500 Hz over about 150 ms, which by all means provides high degree of accuracy.

The signal (information) on the milk amount measured by the infrared sensors, possibly corrected with the contribution of the measurements of the piston position and/or suction pressure and described before, is combined by the signal (information) from the accelerometer to stop the aggregate or provide an alarm or feedback, in case a risk of spillage is imminent.

Risk of spillage exists when the milk in the reservoir approaches the spout or venting hole. Such a risks increases with increasing of the milk amount in the reservoir and with tilting of the breast pump, because the more the milk in the container and/or the more the breast pump is tilted, the closer the milk to the spout or venting hole.

The controller, based on the signals indicative of the milk amount in the reservoir and the tilt angle of the breast pump, generate an alarm or feedback or stop the operation of the aggregate. In this connection if for example the controller implements a lookup table, the lookup table can be indicative of tilt angles and can provide for each tilt angle the maximum allowed milk amount. The below table is an example of a lookup table with angles in the front direction (columns; in this direction the user/mum e.g. bents frontally on the abdomen) and in the transverse direction (rows, in this direction e.g. the mum bents on the hip).

| | | | | | | | | | |
|---|---|---|---|---|---|---|---|---|---|
| | α₁ | α₂ | α₃ | α₄ | α₅ | α₆ | α₇ | α₈ | α₉ |
| β₁ | A_{1,1} | A_{1,2} | A_{1,3} | A_{1,4} | A_{1,5} | A_{1,6} | A_{1,7} | A_{1,8} | A_{1,9} |
| β₂ | A_{2,1} | A_{2,2} | A_{2,3} | A_{2,4} | A_{2,5} | A_{2,6} | A_{2,7} | A_{2,8} | A_{2,9} |
| β₃ | A_{3,1} | A_{3,2} | A_{3,3} | A_{3,4} | A_{3,5} | A_{3,6} | A_{3,7} | A_{3,8} | A_{3,9} |
| β₄ | A_{4,1} | A_{4,2} | A_{4,3} | A_{4,4} | A_{4,5} | A_{4,6} | A_{4,7} | A_{4,8} | A_{4,9} |
| β₅ | A_{5,1} | A_{5,2} | A_{5,3} | A_{5,4} | A_{5,5} | A_{5,6} | A_{5,7} | A_{5,8} | A_{5,9} |
| β₆ | A_{6,1} | A_{6,2} | A_{6,3} | A_{6,4} | A_{6,5} | A_{6,6} | A_{6,7} | A_{6,8} | A_{6,9} |
| β₇ | A_{7,1} | A_{7,2} | A_{7,3} | A_{7,4} | A_{7,5} | A_{7,6} | A_{7,7} | A_{7,8} | A_{7,9} |
| β₈ | A_{8,1} | A_{8,2} | A_{8,3} | A_{8,4} | A_{8,5} | A_{8,6} | A_{8,7} | A_{8,8} | A_{8,9} |
| β₉ | A_{9,1} | A_{9,2} | A_{9,3} | A_{9,4} | A_{9,5} | A_{9,6} | A_{9,7} | A_{9,8} | A_{9,9} |

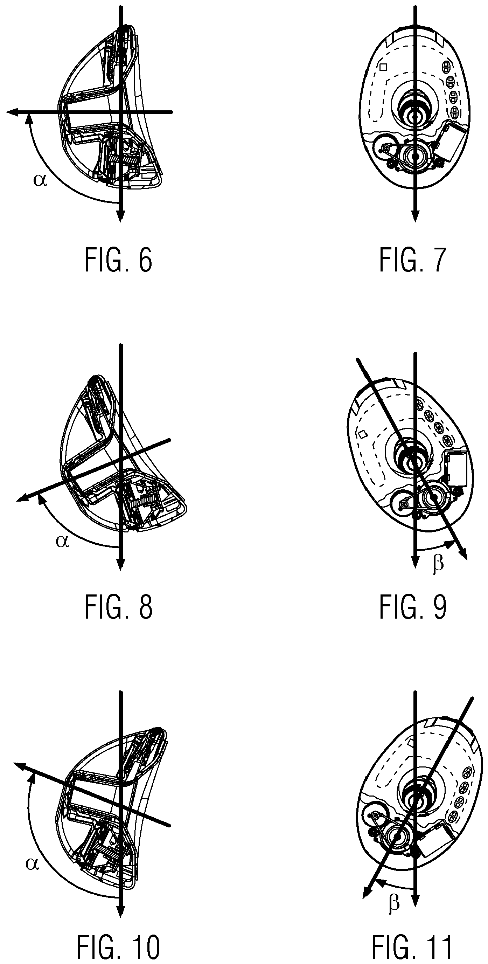

Figures 6 through 11 show the tilt angles of the above table with reference to a vertical axis defined by the force of gravity. Figures 6 and 7 show examples of the angle in the front direction (e.g. alpha (α) in the table; figure 6) equal to 90 degrees and the angle in the transverse direction (e.g. beta (β) in the table; figure 7) equal to 0 degrees; this is a reference position for the breast pump i.e. the "assumed orientation" mentioned above. Figures 8, 9 and figures 10, 11 show the breast pump tilted.

For example, the angles in the table closer to the measured angles can be used for determining the maximum allowed milk amount or the next larger or closer angle in the table with reference to the measured angles can be used for determining the milk amount. For example, is alpha 5 is 90 degrees and alpha 4 is 80 degrees and alpha 6 is 100 degrees, a measured alpha angle equal to 87 degrees is made equal to 80 degrees for the calculation, if the smaller angle next to the measured angle is used; this goes in favour of reliability.

The lookup table provides the maximum allowed milk amount in the reservoir. When the milk amount in the reservoir exceeds the maximum allowed milk amount for the angles measured by the accelerometer, the alarm and/or feedback are generated and/or the aggregate and breast pump operation are stopped. The allowed milk level allowed in the reservoir is much higher than in existing solutions, because it is actually taken into account not only the quantity of milk in the reservoir, but also how close it can come to the spout or venting hole for a given milk quantity.

The signals may be used to stop activation of the aggregate 74 and/or providing an alarm or feedback to the user using the breast pump in case of excessive movement of the breast pump identified by the accelerometer 130, which movement may bring about the risk will spill through the spout 48. Moreover, the calculated yield of milk accumulating in the reservoir may be displayed through the user interface 20 and/or may be used to output a signal indicative of a recommendation to the user to stop the pumping session and replace the filled milk container 14 with a fresh one.

### List of Reference Signs

- 2: breast pump
- 4: housing
- 6: breast shield
- 8: breast shield element
- 10: nipple tunnel
- 12: flange of the breast shield element
- 14: milk container
- 16: bore
- 18: top section
- 20: user interface
- 22: element of a user interface
- 24: optical indicator
- 26: front surface
- 28: rear surface
- 30: free inner end
- 32: two-way valve element
- 34: thread
- 38: container shell element
- 40: lid element
- 42: lid section
- 44: nipple tunnel receptacle
- 46: reservoir
- 48: spout
- 50: spout closure element
- 52: hinge
- 54: membrane
- 56: thread
- 57: membrane working chamber
- 58: trough
- 59: reciprocating suction generating means
- 60: piston
- 62: cap section
- 64: aggregate membrane
- 66: drive housing
- 68: nut element
- 70: drive belt
- 72: drive shaft
- 74: aggregate
- 76: spindle
- 78: roller bearing
- 80: battery
- 82: controller
- 90: pumping chamber
- 92: pumping channel
- 94: outlet space
- 96: closed end
- 98: projection
- 100: flat closing surface
- 102: reflux opening
- 104: reflux valve
- 106: milk outlet opening
- 108: wall section
- 110: milk outlet valve
- 112: container valve
- 120: infrared sensor
- 122: emitter
- 124: receiver
- 126: printed circuit board
- 128: window
- 130: accelerometer
- 132: piston position sensor
- 134: pressure sensor
- 136: contact pads

- A: Air
- L: longitudinal axis
- L1: first fill level
- L2: second fill level
- L3: third fill level
- L4: fourth fill level
- LP: latched piston position
- M: milk
- MF: milk flow
- R: relax phase
- E: expansion phase
- H: holding phase
- RD: reduction phase
- Δp: latched pressure

## Claims

1. A breast pump (2), in particular wearable inside a bra, including a breast shield (6) adapted to at least in part receive a breast of a lactating user, an aggregate (74) for generating suction for expressing milk from the breast, which aggregate (74) comprises a reciprocating suction generating means (59), in particular a piston (60), which reciprocating suction generating means (59) is moved in a reciprocating manner for generating said suction, a milk container (14) providing a reservoir (46) for the expressed milk and a controller (82) operatively connected with the aggregate and provided for controlling the aggregate (74), **characterized by** volume flow assessment means operatively connected with said controller (82) and adapted to estimate the volume flow within the pump (2) on the basis of the movement of said reciprocating suction generating means (59).

2. The breast pump according to claim 1, wherein said controller (82) comprises a latched piston position memory adapted to store a latched piston position (LP), which latched piston position (LP) is indicative of a position of said piston (60), in which the piston (60) is intended to generate suction but which position of the reciprocating suction generating means (59) does not correlate with the expression of milk from the breast, and wherein said volume flow assessment means assesses the volume flow by decreasing a stroke of the reciprocating suction generating means (59) by the latched piston position (LP).

3. The breast pump according to claim 1 or 2, further comprising a pressure sensor (134) adapted to generate a suction pressure signal indicative of a suction pressure generated by the aggregate (74), wherein said controller (82) is operatively connected with said pressure sensor (134) to determine the latched piston position based on the signal of said pressure sensor (134).

4. The breast pump according to claim 3, further comprising a piston position sensor (132) adapted to generate a position signal indicative of a position of the piston (60) and that said controller (82) is operatively connected with said piston position sensor (132) to determine said latched piston position based on said position signal.

5. The breast pump according to any of the preceding claims, further comprising at least one sensor, in particular an infrared sensors (120), being assigned to said reservoir (46), wherein the controller (82) is adapted to assess and output a signal indicative of a milk level on the basis of the volume flow assessment means and signals received from the at least one sensor (120).

6. The breast pump according to claim 6, wherein the plural sensors (120) are provided, which the sensors (120) are spaced apart from each other to provide a signal indicative of a specific milk level within the reservoir (46) and), wherein the controller (82) is adapted to assess and output a signal indicative of an intermediate milk level between two specific milk levels based on information from flow assessment means and signals received from the plural sensors (120).

7. The breast pump according to claim 5 or 6, further comprising an accelerometer (130) and wherein the controller (82) is adapted to assess and output a signal indicative of a milk level based on information from flow assessment means, signals received from the at least one sensor (120) and signals received form the accelerometer (130).

8. The breast pump according to any of the preceding claims, further comprising a pressure sensor (134) adapted to generate a suction pressure signal indicative of a suction pressure generated by the aggregate (74), wherein said controller (82) is operatively connected with piston position sensor (132) and wherein said volume flow assessment means are adapted to estimate the volume flow on the basis of the at least one operational parameter of the aggregate and the suction pressure signal.

9. The breast pump according to claim 8, wherein said volume flow assessment means are adapted to estimate the volume flow on the basis of a suction pressure difference (Δp) sensed by the pressure sensor (134) as the reciprocating suction generating means (59) are held stationary in a suction generating position.

10. The breast pump according to claims 8 or 9, wherein said controller (82) comprises a latched pressure memory adapted to store a latched pressure (Δp), which latched pressure (Δp) is indicative of a pressure difference (Δp) sensed by the pressure sensor (124) at a no milk flow condition.

11. The breast pump according to claim 10, wherein the controller (82) is adapted to assume a no milk flow condition during a stimulation phase, in which the breast is stimulated for the expression of milk.

12. The breast pump according to claim 10 or 11, wherein the controller (82) is adapted to determine a no milk flow condition during an expression phase, in which milk is to be expressed suction from the breast, based on a sequence of sensor signals indicating no milk flow into the reservoir (46).

13. The breast pump according to claim 12, wherein the controller (82) is adapted to periodically determine a no milk flow condition during one pumping session.

14. The breast pump according to any of the preceding claims, further comprising a user interface (20) operatively connected with said controller (82) to output information indicative of the milk level within the reservoir (46) and/or wherein said controller is adapted to control the aggregate (74) based on signals of the volume flow assessment means and/or at least one of the sensors (120) and/or the accelerometer (130) and/or the piston position sensor (132) and/or the pressure sensor (134).
